# EUROPEAN PATENT SPECIFICATION

(11) **EP 3 777 671 B1**
(45) Date of publication and mention of the grant of the patent: **24.12.2025**
(21) Application number: 19776844.3
(22) Date of filing: 14.03.2019
(51) Int. Cl.: H02N 13/00, A61B 5/00, B25J 15/00

(54) **ATTACHMENT PAD**
BEFESTIGUNGSPAD
PASTILLE DE FIXATION

(30) Priority: 29.03.2018 JP 2018065085
(43) Date of publication of application: 17.02.2021
(73) Proprietor: Creative Technology Corporation, Kawasaki-shi, Kanagawa 213-0034 (JP)
(72) Inventor: TATSUMI, Yoshiaki, Kawasaki-shi, Kanagawa 213-0034 (JP); HIRANO, Shinsuke, Kawasaki-shi, Kanagawa 213-0034 (JP); TSUBOI, Kazuki, Kawasaki-shi, Kanagawa 213-0034 (JP); KANEKO, Daiki, Kawasaki-shi, Kanagawa 213-0034 (JP)
(74) Representative: Vossius & Partner Patentanwälte Rechtsanwälte mbB
(86) International application number: PCT/JP2019/010533
(87) International publication number: WO 2019/188341

(56) References cited:
- EP-A1- 2 911 833
- WO-A1-2005/091356
- WO-A1-2011/001978
- WO-A1-2012/090782
- JP-A- 2004 349 663
- US-A1- 2007 223 173
- US-A1- 2010 249 553
- US-A1- 2014 277 739

## Description

### [Technical Field]

The present invention relates to an attachment pad that is used in a state of being attached to human skin utilizing an electrical attachment force.

### [Background Art]

US2010249553A1 describes attachment structures for attaching medical devices to human skin by electro-adhesive forces.

EP 2 911 833 A1 relates to an electroadhesive gripping system including a vacuum- augmented gripper. The gripper includes an electroadhesive surface associated with one or more electrodes and a load-bearing backing structure coupled to the electroadhesive surface.

In recent years, so-called wearable electric products that can be worn on the body and the like have been newly introduced, and motivation for collecting biological data using, for example, various sensors has been increasing. Utilizing a pad having an adhesive means such as an adhesive or a pressure-sensitive adhesive is assumed in such usage applications, but the use of an adhesive, a pressure-sensitive adhesive, or the like takes time and effort for attachment and detachment, and it is disadvantageous in that adhesives cannot be used repeatedly.

Meanwhile, an electrostatic chuck has been conventionally used as means for attaching an attachment target substance by an electrical attachment force. Electrostatic chucks are mainly used for industrial purposes for attaching and holding a semiconductor substrate or a glass substrate when they are treated. An electrostatic chuck generally has a structure in which electrodes are vertically sandwiched by dielectrics, and can attach an attachment target substance with a surface of one of the dielectrics as an attachment surface by application of a voltage to the electrodes according to the attachment principle. In some cases, an electrostatic chuck has heating means, or has a structure in which it is integrally bonded to a metal base having a conduit through which a coolant flows. Furthermore, in addition to such an electrostatic chuck for treating a semiconductor substrate and the like, the inventors of the present application have proposed an electrostatic attachment structure to which the structure and principle of electric attachment of an electrostatic chuck is applied.

For example, PTL 1 proposes an electrostatic attachment structure which is highly convenient in changing its configuration. In the electrostatic attachment structure, multiple sheet members in which two dielectrics are sandwiched between electrodes are laminated. When a voltage is applied between the electrodes, the members can be attached and fixed to each other and exhibited objects and posters such as paper and resin sheets can be attached on the other attachment surface. When application of voltage is cut off after use, sheet members can be easily separated from each other, and attachment target substances can be easily separated. For example, PTL 2 proposes a power generation device which shows good selectivity of installation locations and attachment/detachment properties. The power generation device has attachment and detachment means constituted by surfaces of an electrostatic chuck in which attachment electrodes are sandwiched between two insulating layers and a film-shaped solar cell is attached thereto. The electrostatic attachment structure of PTL 1 and the power generation device of PTL 2 have favorable attachment/detachment properties because the attachment principle of the electrostatic chuck is used. The electrostatic attachment structure and the power generation device are thin and devised considering handleability, However, they are only intended to be attached and fixed to artificial materials that are relatively large and have an even surface such as the above-mentioned industrial products or daily necessities, and their application to attachment target substances such as human skin that is soft and has a certain level of moisture and oil has not yet been studied. In fact, a polyimide film or a polyethylene terephthalate (PET) film is preferably used as a specific dielectric layer (insulator) in the electrostatic attachment structure described in PTL 1 and the power generation device described in PTL 2. However, according to preliminary examinations of the inventors of the present invention, although the detailed cause is not clear, it is presumed that a surface of an electrostatic chuck is reversely charged and an attachment force is thereby decreased in the case of attaching the electrostatic attachment structure (power generation device) to a living body (for example, human skin and the like). Because the occurrence of reverse charging and the decrease in attachment force are more remarkable than in the case of using a semiconductor substrate and the like, it is presumed that moisture and oil in human skin cause the reverse charging and decrease more strongly.

In addition, another reason is that the conventional electrostatic chuck requires a sufficient contact area between a target object and the chuck to obtain a sufficient attachment force, and when both the attachment target side and the chuck side are made flat for increasing the contact area. In this respect, since a surface of a living body (for example, human skin and the like) is generally soft and curved to some extent, it was found that only a part of an attachment surface came into contact with the conventional electrostatic chuck, and therefore a sufficient attachment force could not be obtained.

Furthermore, problems are not limited only to the reverse charging and the contact area. So-called Coulomb force, which is the attachment principle of the conventional electrostatic chuck, is fundamentally weak (several grams per square centimeter), and therefore an effective force that can be used for attachment to a living body cannot be obtained. Accordingly, another stronger force generated at an interface between the chuck surface and the living body, such as the Johnsen-Rahbek effect, is required. As an electrostatic chuck using the Johnsen-Rahbek effect, many chucks formed of a ceramic have been conventionally used, but there are few reports particularly on chucks formed of resins in which followability for securing a contact area with an attachment target object is expected.

Meanwhile, for example, an electrode pad for electrocardiographic measurement as described in PTL 3, or a medical instrument such as a patch for measuring an amount of sweat as described in PTL 4 has been conventionally known as a means used by being attached to human skin, but they are both means in which a pressure sensitive adhesive is used on an attachment surface, and are not means utilizing an electrical attachment force.

### [Citation List]

### [Patent Literature]

[PTL 1] WO 2011/001978
[PTL 2] WO 2012/128147
[PTL 3] Japanese Patent Application Publication No. 2017-113141
[PTL 4] Japanese Patent Application Publication No. 2017-023408

### [Summary of Invention]

### [Technical Problem]

Under such circumstances of a conventional technique, the inventors of the present application have made diligent studies on means that can be attached to substances that are soft and have a certain level of moisture and oil, particularly human skin, by utilizing an electrical attachment force. As a result, the inventors of the present application have found for the first time that an attachment pad can be attached to and held particularly on human skin by an electrical attachment force without using such a conventional pressure sensitive adhesive by configuring a simple attachment pad used together with a power supply device that can receive application of a voltage to an electrode layer, in which a resin film having a specific tensile elastic modulus and a specific volume resistance value is adopted for at least an attachment surface to an attachment target substance, and a laminate sheet in which the resin film and another resin film are sandwiched together between the electrode layer and laminated is obtained, and thus completed the present invention.

Accordingly, an object of the present invention is to provide an attachment pad that can be attached to and held on human skin with favorable followability by utilizing an electrical attachment force.

### [Solution to Problem]

That is, the present invention is defined by the appended claims.

### [Advantageous Effects of Invention]

According to the present invention, it is possible to provide an attachment pad that can be attached to and held on human skin, with favorable followability by utilizing an electrical attachment force. As described above, since the attachment pad can be attached and fixed by an electrical attachment force, it can be used repeatedly without using a chemical adhesive means such as an adhesive or a pressure sensitive adhesive. Furthermore, the attachment pad is simple and cost effective because a release film used therefor becomes unnecessary. In addition, by utilizing the technique of the present invention, it is possible to attach wearable electric products (for example, various sensors that read biometric information, medical devices, ornaments) and the like to surfaces of a living body including human skin and an attachment target substance equivalent to the living body. The technique is expected to expand into fields and industries such as medical care, beauty, sports, clothing, and wearable devices.

### [Brief Description of Drawings]

[Fig. 1]

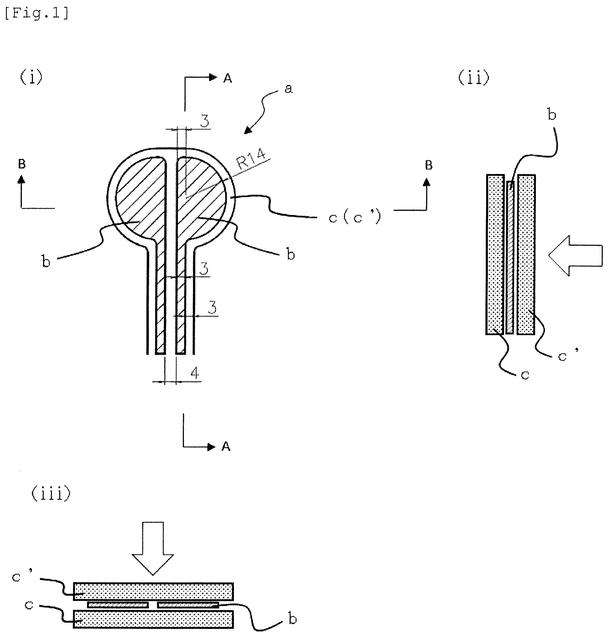

   Fig. 1 is a schematic view illustrating one aspect of a bipolar laminate sheet, (i) is a plan view, (ii) is a cross-sectional explanatory view illustrating a state before lamination in the cross-section of A-A, and (iii) is a cross-sectional explanatory view illustrating a state before lamination in the cross-section of B-B. White arrows in (ii) and (iii) indicate an attachment surface for an attachment target substance.
[Fig. 2]

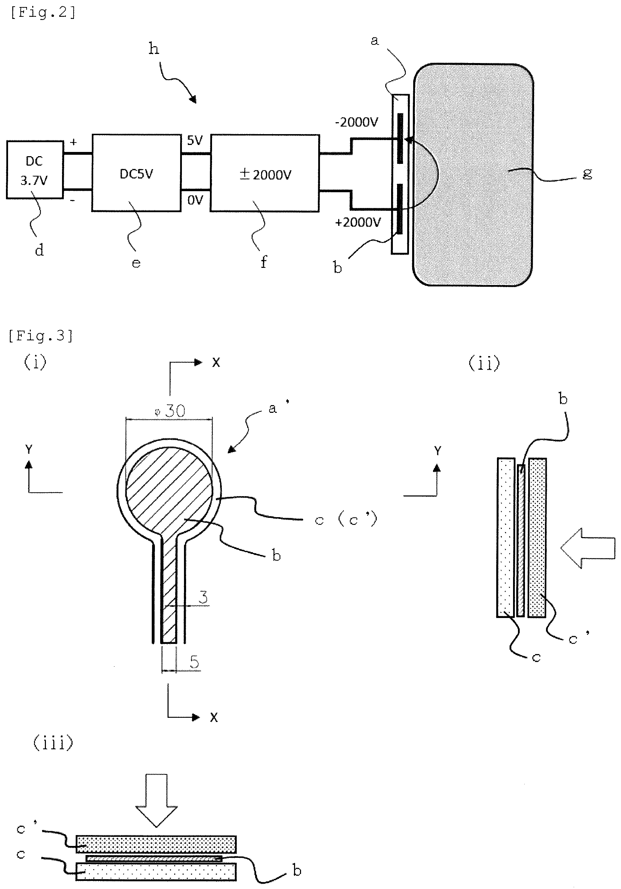

   Fig. 2 is a schematic explanatory view illustrating one embodiment (circuit configuration) in which the bipolar laminate sheet shown in Fig. 1 is used together with a power supply device serving as an attachment pad of the present invention.
[Fig. 3]
   Fig. 3 is a schematic view illustrating a unipolar laminate sheet used in Examples, (i) is a plan view, (ii) is a cross-sectional explanatory view illustrating a state before lamination in the cross-section of X-X, and (iii) is a cross-sectional explanatory view illustrating a state before lamination in the cross-section of Y-Y. White arrows in (ii) and (iii) indicate an attachment surface for an attachment target substance.
[Fig. 4]

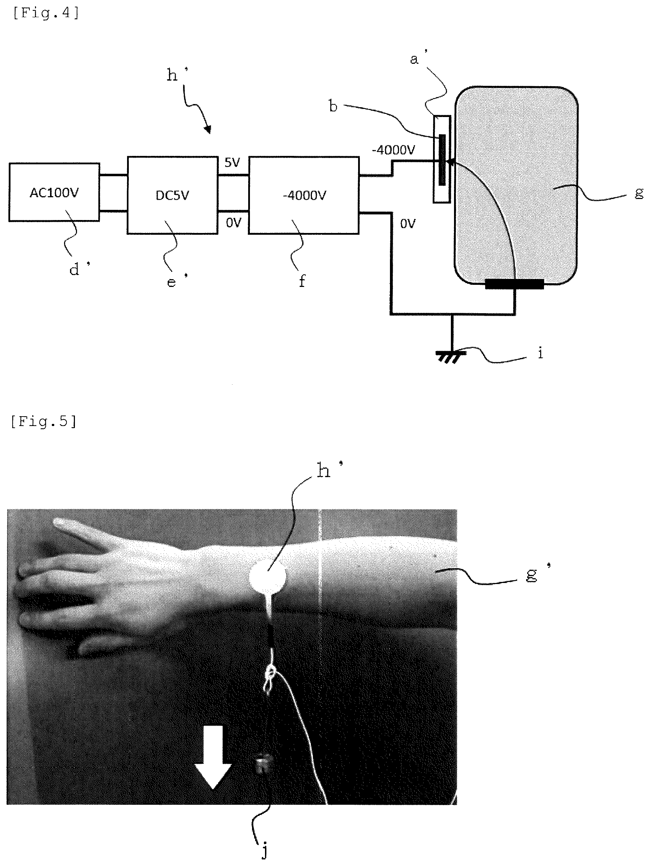

   Fig. 4 is a schematic explanatory view illustrating one embodiment (circuit configuration) in which the unipolar laminate sheet shown in Fig. 3 is used together with a power supply device serving as an attachment pad of the present invention.
[Fig. 5]
   Fig. 5 is an explanatory photograph illustrating a method of evaluating attachment properties of an attachment pad performed in Examples. A white arrow in the figure indicates a vertically downward direction.

### [Description of Embodiments]

Hereinafter, the present invention will be described in detail.

As shown in Figs. 1 to 4, an attachment pad of the present invention at least includes a laminate sheet in which a first resin film, an electrode layer, and a second resin film are sequentially laminated, and the electrode layer is sandwiched between the resin films; and a power supply device that applies a voltage to the electrode layer. In addition, the electrode layer may be a bipolar type having a positive electrode and a negative electrode as shown in Figs. 1 and 2, or may be a unipolar type which has only a positive (negative) electrode and in which a negative (positive) electrode side is grounded as shown in Figs. 3 and 4. Hereinafter, each configuration will be described in detail.

### <Resin film>

Regarding the first and second resin films used in the attachment pad of the present invention, the second resin film is on an attachment surface for human skin in the present invention, and a volume resistivity of at least the second resin film serving as the attachment surface is required to be 1×10¹⁰ to 10¹² Ω·cm. As shown in Examples to be described later, when a volume resistivity of the second resin film on the attachment surface exceeds 1×10¹³ Ω·cm, an attachment force for an attachment target substance (human skin and the like) decreases, and, for example, the attachment pad may fall off even due to its own weight. In contrast, when a volume resistivity is less than 1×10¹⁰ Ω·cm, it is presumed that an attachment force acting on an attachment target substance increases, but this is not preferable because there may be cases in which a small discharge continuously occurs between the attachment pad and the attachment target substance, and itching and pain may be caused especially when the pad is used on a human body, and there may be damage to the skin. A volume resistivity is 1×10¹⁰ to 10¹² Ω·cm in view of both realization of attachment force and safety.

In the present invention, a volume resistivity can be appropriately set regarding the first resin film used on the side opposite to the attachment surface of an attachment target substance, but since there is a probability that an electric current that should flow between the second resin film and an attachment target substance may flow to the first resin film side, a volume resistivity of the first resin film is preferably the same as or higher than a volume resistivity of the second resin film.

In addition, the second resin film serving as the attachment surface is required to have a tensile elastic modulus (Young's modulus) of 1 MPa or more and less than 100 MPa. In particular, in the case of targeting a relatively soft attachment target substance such as human skin, the detailed principle is not clearly identified, but it is required to attach the attachment pad while following the shape of the attachment target substance and to maintain the attached state by suppressing repulsive force (stress) generated inside the attachment pad as much as possible while it is attached to the attachment target substance. Therefore, regarding the resin film, a tensile elastic modulus (Young's modulus) of at least the second resin film serving as the attachment surface is set within the above-mentioned range. A tensile elastic modulus (Young's modulus) of the first resin film used on the side opposite to the attachment surface of the attachment target substance can be appropriately set, but a tensile elastic modulus (Young's modulus) of the first resin film is preferably the same as or smaller than a tensile elastic modulus (Young's modulus) of the second resin film so that flexibility of the entire attachment pad (laminate sheet) is not impaired.

In addition, regarding the resin films, each of the first resin film and the second resin film is required to have a thickness of 20 to 200 µm to ensure insulation properties, followability of attachment to an attachment target substance, and attachment force, where a thickness is preferably 50 to 100 µm. When a thickness is less than 20 µm, dielectric breakdown easily occurs, and the attachment pad may not function as an attachment pad due to pinholes formed in the resin film due to the dielectric breakdown. In contrast, when a thickness exceeds 200 µm, it is not preferable because followability of attachment to an attachment target substance is poor, and a distance to the attachment target substance is large, which may reduce an attachment force.

In addition, the first and second resin films may be the same as or different from each other, and specific examples of such resin films described above include polyimide, polyethylene terephthalate (PET), nylon, polypropylene, polyurethane, soft polyvinyl chloride, polyvinylidene chloride, and the like. Examples thereof further include films processed (by mixing a filler thereinto, and the like) to adjust conductivity thereof. In particular, the second resin film is preferably polyurethane or soft polyvinyl chloride and is more preferably soft polyvinyl chloride so that a volume resistivity and a tensile elastic modulus thereof are set within the above-mentioned predetermined ranges.

### <Electrode layer>

A material, a shape, and the like of the electrode layer used in the present invention are not particularly limited, but a thickness thereof is required to be 1 to 20 µm. When a thickness is less than 1 µm, the electrode layer may be disconnected or conductivity may be reduced due to deformation of the attachment pad. In contrast, when a thickness exceeds 20 µm, hardness of the electrode layer tends to increase, and therefore, flexibility of the entire attachment pad deteriorates, and followability for an attachment target substance may deteriorate. Regarding a material and a production method, for example, a metal foil may be used as it is; an electrode layer obtained by etching a metal film formed by a sputtering method, an ion plating method, or the like into a predetermined shape may be used; or an electrode layer formed into a predetermined shape by spraying a metal material or printing a conductive ink may be used. The shape can be appropriately selected from, for example, a flat plate shape, a semicircular shape, a pattern shape such as a comb shape or a mesh, and the like.

### <Laminate sheet>

In addition, the first and second resin films and the electrode layer described above are used and laminated to form a laminate sheet. The electrode layer is required to be sandwiched between the resin films so that the electrode layer is not exposed. As a specific method, there is a method in which these resin films and electrode layer are sequentially laminated, heat and pressure are applied thereto, and thermoplasticity of the resin films themselves is utilized to fuse the laminate. Alternatively, if necessary, bonding may be performed using a bonding sheet, an adhesive, or a pressure-sensitive adhesive. However, when another material is inserted into an adhesive layer when the attachment pad is deformed or expanded or contracted, because deformation or expansion or contraction may be hindered or peeling of the adhesive surface may occur, the method of fusion welding using thermoplasticity of the films is preferable.

### <Power supply device>

After forming the laminate sheet as described above, a power supply device for applying a voltage to the electrode layers to generate an electrical attachment force is required. The power supply device can be connected to the electrode layer of the laminate sheet via a connection terminal and a switch (both not shown). A power supply device similar to that used for a general electrostatic attachment structure may be used, and any power supply device may be used as long as it can generate a high DC voltage. A potential difference to be generated can be set up to about 500 to 5,000 V, and if necessary, a booster circuit (high voltage generator circuit) capable of boosting to a required voltage may be provided. In particular, it is preferable to design in consideration of the following (a) to (c) to apply the attachment pad of the present invention to the human skin. That is,
(a) Apply as high voltage as possible to generate sufficient attachment force;
(b) Make a potential of a human body as low as 0 V. This is because static electricity will be accumulated inside the human body and the body will be impacted during discharge when a potential applied to the human body is biased to either positive or negative; and
(c) No current flows under any circumstances which may endanger the human body.

Under such design concepts of (a) to (c), it is preferable to determine a voltage applied to the attachment pad in consideration of (b) and (c). In addition, in order to achieve (b), in a case of using a unipolar electrode layer, it is preferable to perform earth-connecting (grounding) connection between an attachment target substance (human skin) and ground of a voltage generation source (for example, a high voltage generator circuit) in a power supply device; whereas in a case of using a bipolar electrode layer, it is preferable to apply positive and negative symmetrical voltages to each of the first electrode and the second electrode in the electrode layer, and as a result, it is preferable that the human body be as low as 0 V. Furthermore, in order to achieve (c), it is preferable to suppress an output current from the power supply to 0.5 mA or less. This is because the human body cannot generally perceive current when it is 0.5 mA or less. However, since a large current may be generated when an electric current is accumulated in the attachment pad or the human body and is discharged at once, an electrostatic capacity of the attachment pad is preferably set to 1,000 pF or less, which is the same level as the human body, specifically to about 10 pF to 100 pF, and a voltage is preferably set within ±5,000 V so that current will be 5 µC or less even when it is accumulated in the human body.

The above-mentioned laminate sheet and power supply device are provided to form the attachment pad of the present invention. If necessary, a sensor or the like may be separately provided to the attachment pad of the present invention. Furthermore, appropriate change or addition of the configuration may be performed within the scope of the object of the present invention such as changing the pattern of the electrode layer.

Regarding an attachment target substance, not only a conductor but also paper or cloth having poor electric conductivity can be adapted. Substances, typically the human skin, that are soft and have a certain level of moisture and oil equivalent to human skin, which are substances having a certain surface to which the attachment pad can be connected and attached (for example, organs, animal skin, plants, foods such as meat and processed meat products/vegetables and processed vegetable products/fruits and processed fruit products, or a combination thereof) can be particularly targeted. In addition, although the principle of attachment is not clear, it is presumed that a minute current is generated between an attachment surface of the attachment pad (laminate sheet) and an attachment target substance, and it is presumed that an attachment force by the Johnsen-Rahbek effect acts between an attachment surface of the attachment pad (laminate sheet) and an attachment target substance.

### [Examples]

Hereinafter, preferable embodiments of the present invention will be specifically described based on examples and comparative examples, but the present invention is not construed as being limited thereto.

### [Production Example 1]

As a bipolar attachment pad, the following was prepared. Two aluminum foils b (thickness: 0.012 mm) as electrode layers having a predetermined shape with dimensions (unit: mm) as shown in Fig. 1 were sandwiched from above and below between two resin films c and c' made from soft polyvinyl chloride [where both had a volume resistivity of 1×10¹⁰ Ω·cm (measured by a method to be described later), a tensile elastic modulus (Young's modulus): 20 to 30 MPa, and a thickness of 100 µm], and they were thermocompression bonded at a temperature of 150°C to obtain a bipolar laminate sheet a.

The produced laminate sheet a was provided with a power supply device as shown in Fig. 2 [a power supply device consisting of a battery d (DC 3.7 V), a DC-DC converter e (5 V output), and a high voltage generator circuit f (±2,000 V output)] to form an attachment pad h according to the present invention. Positive (+2,000 V) and negative (-2,000 V) symmetrical voltages were applied to each of a first electrode and a second electrode in the electrode layer, and thereby it was possible to attach the attachment pad to an attachment target substance (human skin) g while making a potential of the attachment target substance (human body) approximately 0 V.

### [Example 1]

### <Production of attachment pad>

As a unipolar attachment pad, the following was prepared. One aluminum foil b (thickness: 0.012 mm) having a predetermined shape with a dimension (unit: mm) as shown in Fig. 3 as an electrode layer, a soft polyvinyl chloride film c' [volume resistivity: 1×10¹⁰ Ω·cm (measured by the method to be described later), tensile elastic modulus (Young's modulus): 20 to 30 MPa, and thickness 100 µm] as a second resin film serving as an attachment surface of an attachment target substance, a polyimide film c [manufactured by DU PONT-TORAY CO., LTD., trade name: Kapton (registered trademark) H, thickness 25 µm] as a first resin film used on a surface opposite to the attachment surface, and an adhesive layer (not shown) which was made from silicone, had a thickness of 30 µm, and was separately used from the first resin film were all pressure-bonded and laminated to obtain a unipolar laminate sheet a' in which the electrode layer was sandwiched between the first and second resin films.

The laminate sheet a' thus produced was provided with a power supply device as shown in Fig. 4 [a power supply device consisting of a commercial power supply d' (AC 100 V), an AC adapter e' (5 V output), and a high voltage generator circuit f (-4,000 V output)] to form an attachment pad h' according to the present invention. The attachment pad was attached to an attachment target substance (human skin) g. Ground (not shown) of the attachment target substance (human skin) g and the high voltage generator circuit f were earthed.

### <Evaluation of attachment properties of attachment pad>

As shown in Fig. 5, a power supply device was turned on, and the attachment pad h' of Example 1 produced as above was attached to the skin g' of the forearm of a human body (adult man) while pressing the pad with the other hand so that the load was applied vertically downward (the direction of the white arrow in the figure) to generate and hold an electrical attachment force between the attachment pad and the skin of the forearm. A weight j made of brass was installed at a lower end of the attachment pad to check how many grams of the weight the pad could hold, and this was used as evaluation of attachment properties. A holding time of the weight was 15 seconds or longer. The results are shown in Table 1.

**[Table 1]**

| | Second resin film | | | Attachment target substance | Evaluation result |
|---|---|---|---|---|---|
| | Material | Volume resistivity (Ω·cm) | Tensile elastic modulus (MPa) | | |
| Example 1 | Soft polyvinyl chloride | 1×10¹⁰ | 20 to 30 | Human skin | 200 g could be held. |
| Example 2 | | 1×10¹² | 20 to 30 | | |
| Example 3 | | 1×10¹³ | - | | 10 to 20 g could be held. |
| Example 4 | | 1×10¹⁰ | 20 to 30 | Leaf of foliage plant | 10 to 20 g could be held. |
| Example 5 | | 1×10¹⁰ | 20 to 30 | Processed pork meat | 10 to 20 g could be held. |
| Example 6 | | 1×10¹⁰ | 20 to 30 | Vegetable (eggplant) | 10 to 20 g could be held. |
| Comparative Example 1 | | 1×10¹⁵ | - | Human skin | Pad fell off due to its own weight. |
| Comparative Example 2 | Polyimide (Kapton H) | 1×10¹⁷ | 3×10³ | | Attachment force was rapidly reduced and pad fell off due to its own weight. |
| Comparative Example 3 | PET (Lumirror S10) | 1×10¹⁷ | 4×10³ | | |

A volume resistivity of the soft polyvinyl chloride film in Example 1 was measured by a double ring electrode method (IEC 60093, ASTM D257, JIS K 6911, JIS K 6271), and a volume resistivity of each resin film used in Examples 2 to 6 and Comparative Examples 1 to 3 to be described later was also measured by the same method.

### [Example 2]

An attachment pad according to Example 2 was produced in the same manner as in Example 1 except that a soft polyvinyl chloride film (thickness: 100 µm) having a volume resistivity of 1×10¹² Ω·cm (measured by the method described above) and a tensile elastic modulus (Young's modulus) of 20 to 30 MPa was used as the second resin film c' that served as an attachment surface of an attachment target substance. Furthermore, attachment properties of the attachment pad were evaluated in the same manner as in Example 1. The results are as shown in Table 1.

### [Example 3]

An attachment pad according to Example 3 was produced in the same manner as in Example 1 except that a soft polyvinyl chloride film (thickness: 100 µm) having a volume resistivity of 1×10¹³ Ω·cm (measured by the method described above) was used as the second resin film c' that served as an attachment surface of an attachment target substance. Furthermore, attachment properties of the attachment pad were evaluated in the same manner as in Example 1. The results are as shown in Table 1.

### [Example 4]

Attachment properties were evaluated in the same manner as in Example 1 except that the attachment pad h' used in Example 1 was used, the leaf of the foliage plant (plant name: *Ficus umbellata)* was used as the attachment target substance g' instead of the human skin, and the pad was attached to a surface of the leaf. The results are as shown in Table 1.

### [Example 5]

Attachment properties were evaluated in the same manner as in Example 1 except that the attachment pad h' used in Example 1 was used, processed pork meat (manufactured by Foodlier Co., Ltd., product name: loin ham) was used instead of the human skin as the attachment target substance g', and the pad was attached to a surface of the meat after removing some oil on the surface. The results are as shown in Table 1.

### [Example 6]

Attachment properties were evaluated in the same manner as in Example 1 except that the attachment pad h' used in Example 1 was used, vegetable eggplant was used as the attachment target substance g' instead of the human skin, and the pad was attached to a surface of the skin without processing such as cutting. The results are as shown in Table 1.

### [Comparative Example 1]

An attachment pad h' according to Comparative Example 1 was produced in the same manner as in Example 1 except that a soft polyvinyl chloride film (trade name: 711M, manufactured by Nakagawa Chemical Inc., thickness: 100 µm) having a volume resistivity of 1×10¹⁵ Ω·cm (measured by the method described above) was used as the second resin film c' that served as an attachment surface of an attachment target substance. Furthermore, attachment properties of the attachment pad with respect to human skin were evaluated in the same manner as in Example 1. The results are as shown in Table 1.

### [Comparative Example 2]

An attachment pad h' according to Comparative Example 2 was produced in the same manner as in Example 1 except that a polyimide film [manufactured by DU PONT-TORAY CO., LTD., product name: Kapton (registered trademark) H, volume resistivity: 1×10¹⁷ Ω·cm (measured by the method described above), tensile elastic modulus (Young's modulus): 3×10³ MPa, thickness 50 µm] was used as the second resin film c' that served as an attachment surface of an attachment target substance. Furthermore, attachment properties of the attachment pad with respect to human skin were evaluated in the same manner as in Example 1. The results are as shown in Table 1.

### [Comparative Example 3]

An attachment pad h' according to Comparative Example 3 was produced in the same manner as in Example 1 except that a polyethylene terephthalate (PET) film [manufactured by Toray Industries, Inc., product name: Lumirror (registered trademark) S10, volume resistivity: 1×10¹⁷ Ω·cm (measured by the method described above), tensile elastic modulus (Young's modulus): 4×10³ MPa, thickness 50 µm] was used as the second resin film c' that served as an attachment surface of an attachment target substance. Furthermore, attachment properties of the attachment pad with respect to human skin were evaluated in the same manner as in Example 1. The results are as shown in Table 1.

### [Reference Signs List]

- a: Laminate sheet (bipolar type)
- a': Laminate sheet (unipolar type)
- b: Electrode layer
- c: First resin film
- c': Second resin film
- d: Battery
- d': Commercial power supply
- e: DC-DC converter
- e': AC adapter
- f: High voltage generator circuit
- g, g': Attachment target substance (human skin and the like)
- h: Attachment pad (bipolar type)
- h': Attachment pad (unipolar type)
- i: Ground
- j: Weight

## Claims

1. An attachment pad (h, h') for repeated attachment and fixation to human skin (g, g') that is soft and has a certain level of moisture and oil, by utilizing an electrical attachment force, the attachment pad (h, h') comprising:
a laminate sheet(a, a') in which a first resin film (c) having a thickness of 20 to 200 µm, an electrode layer (b) having a thickness of 1 to 20 µm, and a second resin film (c') having a thickness of 20 to 200 µm, which are sequentially laminated and wherein the electrode layer (b) is sandwiched between the resin films (c, c'); and
a power supply device for applying a high DC voltage to the electrode layer (b) to generate an electrical attachment force,
wherein the electrode layer (b) is a bipolar type having a first electrode and a second electrode or is a unipolar type which has only a positive or negative electrode and in which a negative or positive electrode side is, respectively, grounded;
wherein the second resin film (c') is serving as the attachment surface for the human skin (g, g') and has a tensile elastic modulus of 1 MPa or more and less than 100 MPa and a volume resistivity of 1×10¹⁰ to 1×10¹² Ω·cm; and
wherein the attachment pad (h, h') is configured to be attached to the human skin (g, g') onto the attachment surface by the electrical attachment force generated by the voltage application to the electrode layer (b).

2. The attachment pad (h, h') according to claim 1,
wherein the power supply device is configured to provide a potential difference to be generated to be set up to about 500 to 5,000 V; and
wherein in a case of using a unipolar electrode layer an earth-connecting connection between the human skin and ground of a voltage generation source in the power supply device is formed; or
wherein in a case of a bipolar electrode layer positive and negative symmetrical voltages are applies to each of the first electrode and the second electrode in the electrode layer (b).

3. The attachment pad (h, h') according to claim 1 or 2,
wherein the second resin film (c') is made of soft polyvinyl chloride.

4. The attachment pad (h, h') according to any one of claims 1 to 3
wherein the first resin film (c) is made of any one or a combination selected from the group consisting of polyimide, polyethylene terephthalate, nylon, polypropylene, polyurethane, soft polyvinyl chloride, polyvinylidene chloride, and these resins processed.

5. The attachment pad (h, h') according to any one of claims 1 to 4,
wherein the first resin film (c) has a tensile elastic modulus of 1 MPa or more and less than 100 MPa.

6. The attachment pad (h, h') according to any one of claims 1 to 5,
wherein the electrode layer (b) comprises a bipolar electrode having a first electrode and a second electrode.

7. The attachment pad (h, h') according to any one of claims 1 to 5,
wherein the electrode layer (b) comprises a unipolar electrode.

8. An attachment method for attaching the attachment pad (h, h') according to any one of claims 1 to 6 to human skin, the method comprising:
using an electrode layer (b) that comprises a bipolar electrode having a first electrode and a second electrode as an electrode layer, applying a voltage to the electrode layer (b) to attach an attachment pad (h, h') to the human skin while making a potential of the human skin approximately 0 V by applying a positive or negative symmetrical voltage to each of the first electrode and the second electrode.

9. An attachment method for attaching the attachment pad (h, h') according to any one of claims 1 to 5 and 7 to human skin, the method comprising:
using an electrode layer (b) that comprises a unipolar electrode as an electrode layer, applying a voltage to the electrode layer (b) to attach an attachment pad (h, h') to the human skin while making a potential of the human skin approximately 0 V by earth-connecting (grounding) a ground of a voltage generation source in the power supply device with the human skin.

## Patentansprüche

1. Befestigungsauflage (h, h') zur wiederholten Befestigung und Fixierung an menschlicher Haut (g, g'), die weich ist und einen bestimmten Feuchtigkeits- und Ölgehalt aufweist, durch Nutzen einer elektrischen Befestigungskraft, wobei die Befestigungsauflage (h, h') Folgendes umfasst:
eine Laminatfolie (a, a'), in der ein erster Harzfilm (c) mit einer Dicke von 20 bis 200 µm, eine Elektrodenschicht (b) mit einer Dicke von 1 bis 20 µm und ein zweiter Harzfilm (c') mit einer Dicke von 20 bis 200 µm nacheinander laminiert sind und wobei die Elektrodenschicht (b) zwischen den Harzfilmen (c, c') eingebettet ist; und
eine Stromversorgungsvorrichtung zum Anlegen einer hohen Gleichspannung an die Elektrodenschicht (b), um eine elektrische Befestigungskraft zu erzeugen,
wobei die Elektrodenschicht (b) ein bipolarer Typ mit einer ersten Elektrode und einer zweiten Elektrode oder ein unipolarer Typ ist, der nur eine positive oder negative Elektrode aufweist und bei dem jeweils eine negative oder positive Elektrodenseite geerdet ist;
wobei der zweite Harzfilm (c') als Befestigungsfläche für die menschliche Haut (g, g') dient und einen Zugelastizitätsmodul von 1 MPa oder mehr und weniger als 100 MPa sowie einen spezifischen Volumenwiderstand von 1×10¹⁰ bis 1×10¹² Ω·cm aufweist; und
wobei die Befestigungsauflage (h, h') so konfiguriert ist, dass sie durch die elektrische Befestigungskraft, die durch die Spannungsanlegung an die Elektrodenschicht (b) erzeugt wird, an der menschlichen Haut (g, g') auf der Befestigungsfläche befestigt wird.

2. Befestigungsauflage (h, h') nach Anspruch 1,
wobei die Stromversorgungsvorrichtung so konfiguriert ist, dass eine zu erzeugende Potentialdifferenz bereitgestellt ist, die auf etwa 500 bis 5.000 V eingestellt werden kann; und
wobei in einem Fall der Verwendung einer unipolaren Elektrodenschicht eine erdende Verbindung zwischen der menschlichen Haut und dem Erder einer Spannungserzeugungsquelle in der Stromversorgungsvorrichtung ausgebildet wird; oder
wobei in einem Fall einer bipolaren Elektrodenschicht positive und negative symmetrische Spannungen an jede der ersten und zweiten Elektroden in der Elektrodenschicht (b) angelegt werden.

3. Befestigungsauflage (h, h') nach Anspruch 1 oder 2,
wobei der zweite Harzfilm (c) aus weichem Polyvinylchlorid gebildet ist.

4. Befestigungsauflage (h, h') nach einem der Ansprüche 1 bis 3,
wobei der erste Harzfilm (c) aus einem oder einer Kombination gebildet ist, ausgewählt aus der Gruppe bestehend aus Polyimid, Polyethylenterephthalat, Nylon, Polypropylen, Polyurethan, weichem Polyvinylchlorid, Polyvinylidenchlorid und diesen verarbeiteten Harzen.

5. Befestigungsauflage (h, h') nach einem der Ansprüche 1 bis 4,
wobei der erste Harzfilm (c) einen Zugelastizitätsmodul von 1 MPa oder mehr und weniger als 100 MPa aufweist.

6. Befestigungsauflage (h, h') nach einem der Ansprüche 1 bis 5,
wobei die Elektrodenschicht (b) eine bipolare Elektrode mit einer ersten Elektrode und einer zweiten Elektrode umfasst.

7. Befestigungsauflage (h, h') nach einem der Ansprüche 1 bis 5,
wobei die Elektrodenschicht (b) eine unipolare Elektrode umfasst.

8. Befestigungsverfahren zum Befestigen der Befestigungsauflage (h, h') nach einem der Ansprüche 1 bis 6 an menschlicher Haut, wobei das Verfahren Folgendes umfasst:
Verwenden einer Elektrodenschicht (b), die eine bipolare Elektrode mit einer ersten Elektrode und einer zweiten Elektrode als Elektrodenschicht umfasst, Anlegen einer Spannung an die Elektrodenschicht (b), um eine Befestigungsauflage (h, h') an der menschlichen Haut zu befestigen, während ein Potential der menschlichen Haut durch Anlegen einer positiven oder negativen symmetrischen Spannung an jede der ersten Elektrode und der zweiten Elektrode von annähernd 0V erzeugt wird.

9. Befestigungsverfahren zum Befestigen der Befestigungsauflage (h, h') nach einem der Ansprüche 1 bis 5 und 7 an menschlicher Haut, wobei das Verfahren Folgendes umfasst:
Verwenden einer Elektrodenschicht (b), die eine unipolare Elektrode als Elektrodenschicht umfasst, Anlegen einer Spannung an die Elektrodenschicht (b), um eine Befestigungsauflage (h, h') an der menschlichen Haut zu befestigen, während ein Potential der menschlichen Haut bei etwa 0 V durch erdendes Verbinden (Erdung) eines Erders einer Spannungserzeugungsquelle in der Stromversorgungsvorrichtung mit der menschlichen Haut erzeugt wird..

## Revendications

1. Tampon de fixation (h, h') pour une fixation et un attachage répétés à la peau humaine (g, g') qui est souple et présente un degré donné d'humidité et d'huile, en utilisant une force de fixation électrique, le tampon de fixation (h, h') comprenant :
une feuille stratifiée (a, a') dans laquelle un premier film de résine (c) présentant une épaisseur de 20 à 200 µm, une couche d'électrode (b) présentant une épaisseur de 1 à 20 µm, et un deuxième film de résine (c') présentant une épaisseur de 20 à 200 µm sont stratifiés de manière séquentielle et dans lequel la couche d'électrode (b) est prise en sandwich entre les films de résine (c, c') ; et
un dispositif d'alimentation électrique destiné à appliquer une tension CC élevée sur la couche d'électrode (b) pour générer une force de fixation électrique,
dans lequel la couche d'électrode (b) est d'un type bipolaire présentant une première électrode et une seconde électrique ou est un type unipolaire qui présente seulement une électrode positive ou négative et dans lequel un côté d'électrode négative ou positive est respectivement relié à la terre ;
dans lequel le deuxième film de résine (c') fait office de surface de fixation pour la peau humaine (g, g') et présente un module d'élasticité en traction de 1 MPa ou plus et inférieur à 100 MPa et une résistivité volumique de 1×10¹⁰ à 1×10¹² Ω·cm ; et
dans lequel le tampon de fixation (h, h') est configuré pour être fixé à la peau humaine (g, g') sur la surface de fixation par la force de fixation électrique générée par l'application de tension sur la couche d'électrode (b).

2. Tampon de fixation (h, h') selon la revendication 1,
dans lequel le dispositif d'alimentation électrique est configuré pour fournir une différence de potentiel à générer de manière à être établie à environ 500 à 5000 V ; et
dans lequel, dans le cas de l'utilisation d'une couche d'électrode unipolaire, une connexion de connexion à la terre entre la peau humaine et la terre d'une source de génération de tension dans le dispositif d'alimentation électrique est formée ; ou
dans lequel, dans le cas d'une couche d'électrode bipolaire, des tensions symétriques positives et négatives sont appliquées sur chacune de la première et de la seconde électrode dans la couche d'électrode (b).

3. Tampon de fixation (h, h') selon la revendication 1 ou 2,
dans lequel le deuxième film de résine (c') se compose de chlorure de polyvinyle souple.

4. Tampon de fixation (h, h') selon l'une quelconque des revendications 1 à 3,
dans lequel le premier film de résine (c) se compose d'un élément quelconque ou d'une combinaison choisi(e) dans le groupe constitué de polyimide, de polyéthylène téréphtalate, de nylon, de polypropylène, de polyuréthane, de polychlorure de vinyle souple, de polychlorure de vinylidène, et de ces résines traitées.

5. Tampon de fixation (h h') selon l'une quelconque des revendications 1 à 4,
dans lequel le premier film de résine (c) présente un module d'élasticité en traction de 1 MPa ou plus et inférieur à 100 MPa.

6. Tampon de fixation (h, h') selon l'une quelconque des revendications 1 à 5,
dans lequel la couche d'électrode (b) comprend une électrode bipolaire présentant une première électrode et une seconde électrode.

7. Tampon de fixation (h ; h') selon l'une quelconque des revendications 1 à 5,
dans lequel la couche d'électrode (b) comprend une électrode unipolaire.

8. Procédé de fixation destiné à fixer le tampon de fixation (h, h') selon l'une quelconque des revendications 1 à 6 à de la peau humaine, le procédé comprenant :
l'utilisation d'une couche d'électrode (b) qui comprend une électrode bipolaire présentant une première électrode et une seconde électrode comme couche d'électrode, l'application d'une tension sur la couche d'électrode (b) pour fixer un tampon de fixation (h, h') à la peau humaine tout en rendant un potentiel de la peau humaine d'approximativement 0 V en appliquant une tension symétrique positive ou négative sur chacune de la première électrode et de la seconde électrode.

9. Procédé de fixation destiné à fixer le tampon de fixation (h, h') selon l'une quelconque des revendications 1 à 5 et 7 à la peau humaine, le procédé comprenant :
l'utilisation d'une couche d'électrode (b) qui comprend une électrode unipolaire comme couche d'électrode, l'application d'une tension sur la couche d'électrode (b) pour fixer un tampon de fixation (h ; h') sur la peau humaine tout en rendant un potentiel de la peau humaine d'approximativement 0 V en connectant à la terre (mettant à la terre) une terre d'une source de génération de tension dans le dispositif d'alimentation électrique avec la peau humaine.
